# FASCICULE DE BREVET EUROPEEN

(11) **EP 1 120 117 B1**
(45) Date de publication et mention de la délivrance du brevet: **12.09.2007**
(21) Numéro de dépôt: 01400071.5
(22) Date de dépôt: 11.01.2001
(51) Int. Cl.: A61K 31/66

(54) **Procédé pour augmenter la production d'oeufs et consolider la coquille des oeufs chez les volailles**
Verfahren zur Erhöhung der Eierproduktion und Verfestigung von Eierschalen bei Geflügel
Method of increasing the production of eggs and strengthening eggshells in poultry

(30) Priorité: 12.01.2000 FR 0000354
(43) Date de publication de la demande: 01.08.2001
(73) Titulaire: CEVA SANTE ANIMALE, 33500 Libourne (FR)
(72) Inventeur: Bardon, Thierry, 33270 Bouliac (FR); Thibaud, Dominique, 33470 Gujan Mestras (FR)
(74) Mandataire: Bernasconi, Jean Raymond

(56) Documents cités:
- THORP B H ET AL: "THE EFFECT OF A BISPHOSPHONATE ON BONE VOLUME AND EGGSHELL STRUCTURE IN THE HEN" AVIAN PATHOLOGY,HUNTINGDON, CAMBS,GB, vol. 22, no. 4, 1993, pages 671-682, XP000949321 ISSN: 0307-9457
- WILSON S ET AL: "BISPHOSPHONATES: A POTENTIAL ROLE IN THE PREVENTION OF OSTEOPOROSIS IN LAYING HENS" RESEARCH IN VETERINARY SCIENCE,BRITISH VETERINARY ASSOCIATION, LONDON,GB, vol. 64, no. 1, 1998, pages 37-40, XP000949202 ISSN: 0034-5288

## Description

L'invention concerne un procédé pour augmenter la production d'oeufs et consolider la coquille des oeufs chez les volailles.

L'invention concerne par ailleurs un procédé pour la fabrication de préparations administrables à la volaille permettant d'augmenter la production d'oeufs et d'assurer la consolidation des coquilles.

Le procédé de l'invention consiste à administrer au moins un acide bisphosphonique ou un sel physiologiquement acceptable de celui-ci.

Parmi les espèces productrices d'oeufs de consommation, la poule est de loin l'espèce majoritaire. Des progrès constants dans les domaines de la sélection génétique et de la nutrition ont conduit au cours des 30 dernières années à une augmentation régulière du nombre d'oeufs pondus par poule. Il est fréquent désormais qu'une poule ponde plus de 300 oeufs sur une année de ponte ce qui correspond, dans la phase la plus productive de la période de ponte, à un oeuf pondu par jour. Paradoxalement, cette évolution s'est accompagnée d'une réduction de la taille des animaux et donc, en particulier, du volume de la réserve calcique que représente le squelette. Cette réserve est indispensable à la production d'une coquille de qualité. La diminution du calcium disponible dans l'organisme pour produire la coquille a comme première conséquence la production de coquilles plus fragiles présentant des défauts au sein de la trame organique minéralisée. Dans un deuxième temps, si le phénomène persiste, c'est la ponte de l'oeuf qui est compromise.

Les dérivés de l'acide bisphosphonique d'intérêt médical sont aujourd'hui bien connus. Leurs propriétés pharmacologiques et leurs applications thérapeutiques sont bien décrites dans l'état de l'art chez les mammifères, chez l'homme en particulier. Les dérivés de l'acide bisphosphonique, par leurs propriétés anti-résorptives sur l'os et leur action régulatrice du remodelage osseux, font aujourd'hui partie de l'arsenal thérapeutique mis à la disposition du corps médical pour traiter chez l'homme des pathologies associées à des perturbations du métabolisme osseux telles que l'ostéoporose, la maladie de Paget ou les hypercalcémies malignes.

A l'inverse, peu de travaux ont été conduits à ce jour chez les oiseaux. Il apparaît que, chez les oiseaux, les dérivés de l'acide bisphosphonique exercent également leur propriété d'inhibition de la résorption osseuse. Des travaux conduits avec l'alendronate (ou [4-amino-1-hydroxybutylidène] bisphosphonate) chez la poule pondeuse ont en effet démontré l'aptitude de ce composé à limiter la résorption osseuse.

Une première étude rapporte l'effet inhibiteur de l'alendronate sur la résorption osseuse qui accompagne l'acquisition de la maturité sexuelle chez la poule pondeuse lorsque le produit est administré par voie sous-cutanée à la dose de 0,01 mg/kg deux fois par semaine à partir de 16 semaines d'âge et jusqu'à la ponte du premier oeuf (cf Thorp B. H. et al, 1993, Avian Pathol., 22, 671-682).

Une seconde étude utilise un protocole d'administration légèrement différent : l'alendronate est administré par la même voie à la dose de 0,01 mg/kg à raison de 6 administrations au total réparties sur 2 semaines à partir de l'âge de 14 semaines (cf Wilson S. et al, Res. Vet. Sci., 1998, 64, 37-40). Comme la précédente, cette étude démontre l'effet inhibiteur de l'alendronate sur la résorption osseuse qui précède la ponte du premier oeuf.

Les études précédemment rapportées indiquent que l'alendronate n'a pas provoqué de modification de l'âge à l'entrée en ponte.

L'usage de composés susceptibles de réduire la mobilisation calcique nécessaire à la production de la coquille de l'oeuf paraît néfaste à la fois pour la production de coquilles de qualité et pour la production d'oeufs. Les dérivés de l'acide bisphosphonique, par leur action anti-résorptive, doivent a priori exercer de tels effets néfastes.

Dans les deux études précédemment rapportées, il a été montré que le volume de l'os médullaire est sensiblement moins important chez les poules recevant l'alendronate comparativement aux poules témoins et ceci quel que soit le moment d'administration du composé, c'est-à-dire avant l'entrée en ponte ou en cours de ponte. L'os médullaire est un os propre aux oiseaux femelles ; cet os agit comme un réservoir de calcium nécessaire à la constitution de la coquille de l'oeuf. A chaque cycle de ponte, cet os subit une résorption partielle permettant la libération du calcium puis une nouvelle minéralisation qui sera exploitée au cycle suivant. Les résultats obtenus sur la diminution du volume de l'os médullaire dans les études précédemment citées suggèrent donc que l'administration des dérivés de l'acide bisphosphonique avant l'entrée en ponte ou en cours de ponte est susceptible de compromettre la minéralisation de la coquille de l'oeuf et, par voie de conséquence, la qualité de la coquille des oeufs.

L'étude de Thorp et al. démontre par ailleurs que l'administration d'alendronate en cours de ponte aux doses de 0,01, 0,1 ou 1 mg/kg peut entrainer une réduction, voire un arrêt, de la ponte. Ainsi, l'injection d'une dose de 1 mg/kg par voie sous-cutanée tous les 2 jours pendant 2 semaines, environ 18 semaines après l'entrée en ponte, provoque un arrêt complet de ponte en quelques jours. De plus, le produit est à l'origine d'une altération de la qualité de la coquille, d'autant plus importante que la dose est élevée.

Or, les présents inventeurs ont découvert de façon étonnante que les composés de type bisphosphonates sont utiles pour augmenter la production d'oeufs chez la volaille ainsi que pour améliorer la qualité de la coquille des oeufs pondus.

Plus précisément, l'invention concerne un procédé pour augmenter la production d'oeufs et consolider la coquille des oeufs chez les volailles, comprenant l'administration à l'animal d'au moins un composé bisphosphonique choisi parmi un acide bisphosphonique, un sel physiologiquement acceptable de celui-ci ,leurs hydrates et leurs mélanges, et ceci avant l'âge moyen d'entrée en ponte.

Par acide bisphosphonique et sel de cet acide, on entend généralement un composé présentant l'enchaînement P-C-P.

Les sels de ce composé avec des acides ou des bases minéraux ou organiques pharmaceutiquement acceptables sont également utilisables dans le cadre de l'invention. Des exemples de sels avec des acides sont les chlorhydrate, bromhydrate, sulfate, acétate, hydrogénosulfate, dihydrogénophosphate, méthanesulfonate, méthylsulfate, maléate, fumarate, sulfonate, 2-naphtalènesufonate, glycolate, gluconate, citrate, iséthionate, benzoate, salicylate, ascorbate, tartrate, succinate, lactate, glutarate, toluènesulfonate et ascorbate. Comme exemple de sels avec des bases minérales ou organiques, on peut citer les sels d'ammonium ou les sels de métaux alcalins, tels que par exemple les sels de sodium.

Les hydrates de ces composés sont de même utilisables selon l'invention.

Les composés de type bisphosphonique décrits dans la technique comme agent favorisant la résorption osseuse ou préconisés dans le traitement de la maladie de Paget sont utilisables dans le cadre de l'invention. Plus généralement, les acides bisphosphoniques et les sels de ces acides décrits dans les demandes de brevets suivants entrent dans le cadre de la définition du composé bisphosphonique administrable selon l'invention : WO 87/03598, EP 325 482, BE 822 930 ; EP 304 961 ; US 4 621 077 ; FR 2 826 223 ; WO 86/00902 ; EP 162 510 ; EP 186 405 ; US 4 922 007 ; US 4 578 376 ; BE 865 434 ; US 4 134 969 ; DE 2 130 794 et BE 902 308.

De manière générale, le composé bisphosphonique a pour formule : 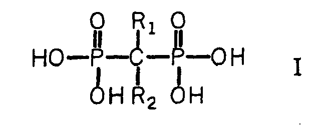
dans laquelle R₁ et R₂ sont indépendamment un atome d'hydrogène ; un atome d'halogène ; un groupe hydroxy ; un groupe -T ; ou un groupe -XT ;
T est choisi parmi un radical hydrocarboné aliphatique saturé ou insaturé, éventuellement substitué et/ou éventuellement interrompu par un ou plusieurs O, S, N, N-CO, CO-N, CO, SO ou SO₂ ; un radical carbocyclique ou hétérocyclique, saturé, insaturé ou aromatique, éventuellement substitué ; un radical présentant à la fois une partie aliphatique telle que définie ci-dessus et une partie carbocyclique et/ou hétérocyclique telle que définie ci-dessus, ledit radical étant éventuellement substitué et/ou éventuellement interrompu par un plusieurs O, S, N, N-CO, CO-N, CO, SO ou SO₂ ;
X est choisi parmi O, NH, NT, S, CO, CO-NT, NT-CO où T est tel que défini ci-dessus ;
ou est un sel physiologiquement acceptable de ce composé, ou encore un de leurs hydrates.

Par radical aliphatique, on entend un radical hydrocarboné non cyclique, tel que alkyle, alcényle et alcynyle, ces derniers étant une ou plusieurs fois insaturés.

Des exemples de groupes alkyle sont les radicaux méthyle, éthyle, propyle, isopropyle, butyle, isobutyle, t-butyle, pentyle, isopentyle, néopentyle, 2-méthylbutyle, 1-éthylpropyle, hexyle, isohexyle, néohexyle, 1-methylpentyle, 3-méthylpentyle, 1,1-diméthylbutyle, 1,3-diméthylbutyle, 2-éthylbutyle, 1-méthyl-1-éthylpropyle, heptyle, 1-méthylhexyle, 1-propylbutyle, 4,4diméthylpentyle, octyle, 1-méthylheptyle, 2-éthylhexyle, 5,5-diméthylhexyle, nonyle, décyle, 1-méthylnonyle, 3,7-diméthyloctyle et 7,7-diméthyloctyle.

Les radicaux alkyle peuvent présenter jusqu'à 25 atomes de carbone. Ils contiennent de préférence de 1 à 12 atomes de carbone, mieux encore de 1 à 6 atomes de carbone.

Les groupes alcényle et alcynyle peuvent présenter jusqu'à 25 atomes de carbone. Ils comprennent de préférence de 2 à 12 atomes de carbone, mieux encore de 2 à 6 atomes de carbone.

Par radical carbocyclique, on entend un radical hydrocarboné monocyclique ou polycyclique, de préférence monocyclique ou bicyclique. Généralement, ce radical comprend 3 à 18 atomes de carbone (par exemple de 3 à 10).

De tels radicaux saturés ou insaturés sont par exemple cycloalkyle et cycloalcényle. Cycloalcényle peut comprendre une ou plusieurs insaturations. Des exemples de cycloalkyle sont cyclopentyle, cyclohexyle, cycloheptyle, cyclooctyle, adamantyle ou norbornyle.

Les radicaux carbocycliques aromatiques sont par exemple les radicaux aryle, mono- ou bicycliques, de préférence en C₆-C₁₈.

Des exemples en sont les radicaux phényle et naphtyle, anthryle et phénanthryle, plus préférablement phényle.

Par radical hétérocyclique, on entend des radicaux monocycliques ou polycycliques, de préférence monocycliques ou bicycliques, comprenant 1 ou plusieurs hétéroatomes choisis parmi O, N et S. Ces radicaux sont constitués de noyaux cycliques condensés deux à deux ou reliés, deux à deux, par des liaisons sigma, chaque noyau présentant ou non une ou plusieurs insaturations et présentant de préférence 5 à 10 chaînons, mieux encore de 5 à 8. On distingue les radicaux hétérocycliques aromatiques, des radicaux hétérocycliques saturés et insaturés.

De manière préférée, le radical hétérocyclique comprend 1, 2, 3 ou 4 hétéroatomes choisis parmi O, N et S.

Des exemples en sont les radicaux pyrrolidine, dioxolane, imidazolidine, pyrazolidine, pipéridine, dioxane, morpholine, dithiane, thiomorpholine, pipérazine, trithiane, indoline, indène, carbazole, phénothiazine, phénoxazine, fluorène, leurs dérivés saturés, insaturés et aromatiques, ainsi que les hétéroaryles définis ci-après : furyle, thiényle, pyrrolyle, oxazolyle, thiazolyle, imidazolyle, pyrazolyle, isoxazolyle, isothiazolyle, oxadiazolyle, triazolyle, thiadiazolyle, pyridyle, pyridazinyle, pyrazinyle, triazinyle, indolizinyle, indolyle, isoindolyle, benzofuryle, benzothiényle, indazolyle, benzimidazolyle, benzothiazolyle, purinyle, quinolizinyle, quinoléinyle, isoquinoléinyle, cinnolinyle, phtalazinyle, quinazolinyle, quinoxalinyle, naphthyridinyle, ptéridinyle, acridinyle, phénazinyle, leurs dérivés saturés ou insaturés.

Les radicaux présentant à la fois une partie aliphatique et une partie carbocyclique et/ou hétérocyclique ont avantageusement pour formule -alk-Ar dans laquelle alk est alkyle, de préférence en C₁-C₁₂ et Ar représente (C₆-C₁₈)aryle ou hétéroaryle mono-, bi- ou tricyclique présentant de 1 à 4 hétéroatomes choisis parmi O, N et S. Un exemple préféré de tel radical est benzyle.

Lorsque Ar représente hétéroaryle, il est de préférence choisi parmi les hétéroaryles énumérés ci-dessus.

Par atome d'halogène, on entend généralement un atome de chlore, de brome et d'iode ou de fluor.

Lorsque T représente un radical hydrocarboné aliphatique ou un radical présentant à la fois une partie aliphatique et une partie carbocyclique et/ou hétérocyclique, ledit radical T peut être éventuellement interrompu par un ou plusieurs O, S, N, N-CO, CO-N, CO, SO ou SO₂.

Lorsqu'un radical aliphatique est interrompu par N, N-CO et CO-N, l'atome d'azote porte en outre un atome d'hydrogène ou un radical aliphatique tel que défini ci-dessus pour T. Lorsque le radical interrompu par N, N-CO ou CO-N est un radical présentant à la fois une partie aliphatique et une partie hétérocyclique ou carbocyclique, l'atome d'azote porte un radical carbocyclique ou hétérocyclique, ou bien un radical aliphatique ou bien encore un atome d'hydrogène.

De manière préférée, X est choisi parmi O, S, NH et NT, mieux encore parmi O et S.

Lorsque T représente un radical hydrocarboné aliphatique (interrompu ou non par O, S, N, N-CO, CO-N, CO, SO ou SO₂) ; un radical carbocyclique ou hétérocyclique ; ou un radical présentant à la fois une partie aliphatique et une partie carbocyclique et/ou hétérocyclique (interrompu ou non par O, S, N, N-CO, CO-N, CO, SO ou SO₂), ledit radical T peut être éventuellement substitué.

Selon l'invention, le substituant du radical représentant T n'est pas critique. Il s'agit de façon générale d'un groupe organique monovalent. A titre de substituants préférés, on peut mentionner les groupes -OH, SH et -NH₂ et les atomes d'halogène, plus préférablement les groupes -OH, SH et les atomes d'halogène.

De façon avantageuse, T est choisi parmi (C₁-C₁₂)alkyle éventuellement substitué par un ou plusieurs groupes K tels que définis ci-dessous ; (C₆-C₁₈)aryle mono-, bi- ou tricyclique éventuellement substitué par un ou plusieurs groupes K ; hétéroaryle mono-, bi- ou tricyclique comprenant 1, 2, 3 ou 4 hétéroatomes choisis parmi O, N et S éventuellement substitué par un ou plusieurs groupes K, et dans lequel chaque unité monocyclique présente de 5 à 8 chaînons ; (C₆-C₁₈)aryl-(C₁-C₁₂)alkyle éventuellement substitué par un ou plusieurs groupes K ; hétéroaryl(C₁-C₁₂)alkyle éventuellement substitué par un ou plusieurs groupes K où hétéroaryle est tel que défini ci-dessus ; hétérocycle saturé monocyclique présentant de 5 à 8 chaînons et 1, 2, 3 ou 4 hétéroatomes choisis parmi N, S et O, éventuellement condensé à un noyau (C₆-C₁₈)aryle tel que défini ci-dessus, et éventuellement substitué par un ou plusieurs groupes K ; ou (C₁-C₁₂)alkyle substitué par hétérocycle saturé monocyclique tel que défini ci-dessus éventuellement condensé à (C₆-C₁₈)aryle et éventuellement substitué sur la partie alkyle, aryle ou sur la partie hétérocycle par un ou plusieurs groupes K ;

K est choisi parmi OH ; SH ; (C₁-C₁₂)alcoxy; (C₁-C₁₂)alkylthio ; -NH₂ ; (C₁-C₁₂)alkylamino ; di(C₁-C₁₂)alkylamino ; et un atome d'halogène.

De préférence, K, est choisi parmi -OH ; -SH ; (C₁-C₁₂)alcoxy ; (C₁-C₁₂)alkylthio ; (C₁-C₁₂)alkylamino ; di(C₁-C₁₂)alkylamino ; et un atome d'halogène. Mieux encore, K est choisi parmi -OH ; -SH ; (C₁-C₁₂)alcoxy ; (C₁-C₁₂)alkylthio ; et un atome d'halogène.

Un sous-groupe préféré des composés bisphosphoniques décrits ci-dessus est celui défini par la formule I ci-dessus dans laquelle
- R₁ représente un atome d'hydrogène, un atome d'halogène, un hydroxyle, un amino, un monoalkyl(C₁-C₄)amino, un dialkyl(C₁-C₄)amino ;
- R₂ représente un atome d'halogène, un alkyle linéaire comportant de 1 à 5 atomes de carbone non substitué ou substitué par un groupe choisi parmi un atome de chlore, un hydroxyle, un amino, un monoalkyl(C₁-C₄)amino, un dialkyl(C₁-C₄)amino ; un cycloalkyl(C₃-C₇)amino,
ou R₂ représente un phénoxy, un phényle, un thiol, un phénylthio, un chlorophénylthio, un pyridyle, un pyridylméthyle, un 1-pyridyl-1-hydroxyméthyle, un imidazolylméthyle, un thiomorpholin-4-yle.

Parmi ces composés, on préfère que R₂ soit choisi parmi un atome d'halogène, un alkyle linéaire comportant de 1 à 5 atomes de carbone, non substitué ou substitué par un groupe choisi parmi un atome de chlore ; un groupe hydroxyle ; un mono(C₁-C₄)alkylamino ; un dialkyl(C₁-C₄)amino; et un cycloalkyl(C₃-C₇)amino.

Mieux encore, on préfère que R₂ soit choisi parmi un atome d'halogène, un alkyle linéaire comportant de 1 à 5 atomes de carbone, non substitué ou substitué par un groupe choisi parmi un atome de chlore ; un groupe hydroxyle ; et un cycloalkyl(C₃-C₇)amino.

Un second sous-groupe préféré est constitué des composés de formule I dans laquelle :
- R₁ représente un atome d'hydrogène ; un atome d'halogène ; un groupe hydroxy éventuellement substitué par un groupe Aₒ ; un groupe Aₒ ; un groupe alkyle, de préférence en C₁-C₁₂, éventuellement substitué par un ou plusieurs substituants indépendamment choisis parmi un atome d'halogène, hydroxy, hydroxy substitué par un groupe Bₒ, thiol, thiol substitué par un groupe Bₒ et les groupes Aₒ ;
- R₂ représente un groupe thiol éventuellement substitué par Aₒ ou par un groupe alkyle, de préférence en C₁-C₁₂, lui-même éventuellement substitué par un ou plusieurs substituants choisis parmi hydroxy, un atome d'halogène, un groupe Aₒ et un groupe thiol ;
- Aₒ représente un groupe aryle ; hétéroaryle ; arylalkyle ; hétéroarylalkyle ; un groupe hétérocyclique saturé monocyclique éventuellement condensé à (C₆-C₁₈)aryle ; ou un groupe alkyle substitué par un tel groupe hétérocyclique saturé monocyclique éventuellement condensé à (C₆-C₁₈)aryle ; chacun de ces groupes étant éventuellement substitué sur la partie aromatique, hétérocyclique ou sur la partie aliphatique par un ou plusieurs hydroxy, halogène, alkyle (de préférence en C₁-C₁₂), alcoxy (de préférence en C₁-C₁₂), thiol ou alkylthio (de préférence en C₁-C₁₂);
- Bₒ représente Aₒ ou alkyle, de préférence en C₁-C₁₂, éventuellement substitué par un ou plusieurs groupes choisis parmi hydroxy, halogène, alcoxy (de préférence en C₁-C₁₂), thiol ou alkylthio (de préférence en C₁-C₁₂).

Dans ce sous-groupe, hétérocycle, hétéroaryle et aryle sont généralement tels que définis ci-dessus.

De préférence, hétéroaryle désigne pyridyle, thiényle ou furyle et aryle représente phényle. De préférence, hétérocycle désigne pyrrolidine, tétrahydrofuryle, tétrahydrothiényle, pipéridine ou morpholine.

Parmi ces composés, on préfère tout particulièrement les composés de formule I dans laquelle :
- R₁ est un atome d'halogène, de préférence le chlore ou un groupe (C₁-C₁₂)alkyle éventuellement substitué, de préférence par un ou plusieurs atomes d'halogène ;
- R₂ est un groupe (C₆-C₁₂)arylthio ou (C₄-C₁₀)hétéroarylthio, chacun de ces groupes étant éventuellement substitué, de préférence par un ou plusieurs groupes thiol et/ou halogène.

Parmi les composés bisphosphoniques utilisables selon l'invention, ceux appartenant à l'un des groupes E1 à E3 suivants se distinguent notamment de par leur mode d'action cellulaire différent.

Groupe E1 : composés de formule I dans laquelle R₁ et/ou R₂ représentent un radical hydrocarboné aliphatique, saturé ou insaturé, portant en position terminale un groupe amino non substitué.

Groupe E2 : composés de formule I dans laquelle R₁ et/ou R₂ représentent un radical hydrocarboné aliphatique, saturé ou insaturé, portant un substituant amino substitué ou non (en position terminale ou non).

Groupe E3 : composés de formule I dans laquelle R₁ et/ou R₂ comprennent un ou plusieurs atomes d'azote.

Parmi les composés bisphosphoniques utilisables dans le cadre de l'invention, on peut mentionner :
- l'acide 2-pyridin-2-yl-éthylidène bisphosphonique dont la dénomination commune internationale est l'acide piridronique, et ses sels de sodium;
- l'acide 3-amino-1-hydroxy-propylidène bisphosphonique dont la dénomination commune internationale est l'acide pamidronique, et ses sels de sodium;
- l'acide 3-(diméthylamino)-1-hydroxypropylidène bisphosphonique dont la dénomination commune internationale est l'acide olpadronique, et ses sels ;
- l'acide 1-hydroxy-3-(méthylpentylamino)propylidène bisphosphonique dont la dénomination commune internationale est l'acide ibandronique, et ses sels ;
- l'acide 4-amino-1-hydroxy-butylidène bisphosphonique dont la dénomination commune internationale est l'acide alendronique, et ses sels de sodium;
- l'acide 6-amino-1-hydroxy-hexylidène bisphosphonique dont la dénomination commune internationale est l'acide néridronique, et ses sels;
- l'acide 1-hydroxy-2-(pyridin-3-yl)éthylidène bisphosphonique dont la dénomination commune internationale est acide risédronique et ses sels de sodium;
- l'acide 1-hydroxy-2-(1H-imidazol-1-yl)éthylidène bisphosphonique dont la dénomination commune internationale est l'acide zolédronique, et ses sels;
- l'acide (cycloheptylamino)méthylène bisphosphonique dont la dénomination commune internationale est l'acide incadronique et ses sels;
- l'acide 2-hydroxyéthylidène-2-(pyridin-3-yl)-1,1- bisphosphonique et ses sels de sodium.

Des composés bisphosphoniques particulièrement préférés sont :
- l'acide dichlorométhylène bisphosphonique dont la dénomination commune internationale est l'acide clodronique, et ses sels de sodium;
- l'acide 1-hydroxy-éthylidène bisphosphonique dont la dénomination commune internationale est l'acide étidronique, et ses sels de sodium ;
- l'acide phénoxyméthylène bisphosphonique et ses sels;
- l'acide thiomorpholinométhylène bisphosphonique et ses sels;
- l'acide 4-chloro-phénylthiométhylène bisphosphonique dont la dénomination commune internationale est acide tiludronique et ses sels pharmaceutiquement acceptables, notamment le sel disodique;

De façon générale, on distingue les composés bisphosphoniques azotés, des composés bisphosphoniques non azotés, ces derniers étant préférés.

Selon la présente invention, l'usage de l'acide tiludronique et de ses sels pharmaceutiquement acceptables, en particulier le sel disodique, ou de ses hydrates est particulièrement préféré.

Il doit être entendu que selon l'invention, l'administration d'un mélange de deux composés bisphosphoniques ou plus est envisageable.

Le procédé de l'invention est plus spécifiquement approprié à l'administration de composés bisphosphoniques chez les volailles productrices d'oeufs propres à la consommation, telles que les poules, les canards et les cailles.

Selon un mode de réalisation préféré, la volaille est une poule.

La voie d'administration peut être la voie orale, la voie parentérale ou la voie nasale. Par voie orale, le traitement peut être administré dans l'eau de boisson. Par voie parentérale, on peut utiliser la voie sous-cutanée, intradermique, intramusculaire, intraveineuse ou intra-articulaire. Par voie nasale, le traitement peut être administré au moyen de dispositifs assurant la dispersion dans l'air de fines gouttelettes de liquide, préférentiellement d'eau, dans lequel aura été préalablement incorporé le médicament. De tels dispositifs sont par exemple des nébuliseurs, des atomiseurs, des vaporisateurs, des aérosols.

La forme galénique d'administration du médicament est fonction de la voie d'administration. Par la voie orale, des formes solubilisables dans l'eau de boisson sont préférées. Parmi celles-ci, on peut citer des poudres orales, des comprimés à dissolution rapide, des comprimés effervescents, des solutions buvables. Pour la voie parentérale, le traitement peut être administré sous la forme de solution, préférentiellement aqueuse, de suspension, d'implants ou de préparations lyophilisées.

Les préparations destinées à la voie orale peuvent contenir, en plus du ou des composés bisphosphoniques, un agent de désintégration, un agent d'écoulement, un lubrifiant et tout excipient de masse convenable.

Comme excipient de masse, on peut utiliser le lactose, la cellulose ou les amidons. Comme lubrifiant, on peut utiliser l'acide stéarique, le stéarate de magnésium, la L-leucine ou, par exemple, le tribéhénate de glycérol. Comme agent de désintégration, on peut utiliser le carboxyméthylamidon sodique, la carboxyméthylcellulose sodique réticulée ou, par exemple, la polyvinylpyrrolidone réticulée. Comme agent d'écoulement, on peut utiliser la silice pure ou le dioxyde de silicium colloïdal.

La présente invention se réfère également aux formes orales à dissolution instantanée et aux formes orales effervescentes obtenues en ajoutant un couple effervescent à la composition selon l'invention. Comme couple effervescent, on peut utiliser, l'acide tartrique et le bicarbonate de sodium ou l'acide citrique et le bicarbonate de sodium.

L'invention se réfère également aux comprimés à dissolution instantanée, aux comprimés effervescents ainsi qu'aux comprimés recouverts d'un enrobage. Une composition contenant du laurylsulfate de sodium selon le brevet européen EP 336851 est particulièrement convenable.

Les préparations injectables sont préparées par mélange d'un ou plusieurs dérivés de l'acide bisphosphonique avec un régulateur de pH, un agent tampon, un agent de mise en suspension, un agent de solubilisation, un stabilisant, un agent de tonicité et/ou un conservateur, et par transformation du mélange en une injection intraveineuse, sous-cutanée, intramusculaire, intradermique ou intra-articulaire selon un procédé classique. Le cas échéant, les préparations injectables peuvent être lyophilisées selon un procédé classique.

Des exemples d'agents de mise en suspension englobent la méthylcellulose, le polysorbate 80, l'hydroxyéthylcellulose, l'acacia, la gomme adragante en poudre, la carboxyméthycellulose sodique et le monolaurate de sorbitane polyéthoxylé.

Des exemples d'agent de solubilisation englobent l'huile de ricin solidifiée par du polyoxyéthylène, le polysorbate 80, le nicotinamide, le monolaurate de sorbitane polyéthoxylé, le macrogol et l'ester éthylique d'acide gras d'huile de ricin.

En outre, le stabilisant englobe le sulfite de sodium, le métalsulfite de sodium et l'éther, tandis que le conservateur englobe le p-hydroxybenzoate de méthyle, le p-hydroxybenzoate d'éthyle, l'acide sorbique, le phénol le crésol et le chlorocrésol.

Un exemple d'agent de tonicité est le mannitol.

Lors de la préparation des solutions ou suspensions injectables, il est souhaitable de veiller à ce qu'elles soient isotoniques au sang.

Selon un autre de ses aspects, l'invention concerne l'utilisation d'au moins un composé bisphosphonique choisi parmi un acide bisphosphonique, un sel physiologiquement acceptable de celui-ci et leurs hydrates pour la préparation d'une formulation permettant une augmentation de la production d'oeufs et une consolidation de la coquille des oeufs pondus.

Les composés bisphosphoniques ont la double fonction d'améliorer la qualité, et notamment les propriétés mécaniques de résistance des coquilles des oeufs pondus, et d'augmenter la productivité des volailles.

Il a été démontré que l'administration de composés bisphosphoniques chez la volaille entraine une augmentation du nombre total d'oeufs pondus au cours de la période de ponte, ainsi qu'une augmentation du nombre moyen d'oeufs pondus par semaine pendant la période de ponte.

Le tracé de courbes de ponte permet une visualisation des effets obtenus ; la courbe de ponte représente les variations du nombre moyen d'oeufs pondus par semaine et par poule en fonction du temps, exprimé en semaine. En variante, on peut représenter les variations, en fonction du temps, du taux de ponte, le taux de ponte étant défini comme le rapport du nombre moyen (n) d'oeufs pondus par poule et par semaine à nₒ (qui est la valeur théorique correspondant à un oeuf pondu par jour et par poule) multiplié par 100. En l'occurrence nₒ vaut 7.

Les effets positifs obtenus se manifestent par une pente plus faible de la courbe de ponte et un pic de ponte (nombre maximum d'oeufs pondus ou taux maximum) plus élevé.

Parallèlement, on observe une résistance mécanique supérieure de la coquille suite à l'administration des composés bisphosphoniques.

De manière avantageuse, l'administration des composés bisphosphoniques est initiée dans les deux mois précédant l'âge moyen d'entrée en ponte, de préférence dans les 6 semaines précédant l'âge moyen d'entrée en ponte.

La durée de traitement est fonction de la voie d'administration. Les composés bisphosphoniques peuvent être administrés en une seule fois ou à intervalles (de préférence à intervalles réguliers) en l'espace de quelques jours ou de quelques semaines.

De manière préférée, l'administration des composés bisphophoniques est stoppée dès l'entrée en ponte.

Le rythme d'administration doit également être choisi en fonction de la voie d'administration, de la nature du dérivé de l'acide bisphosphonique et de la dose.

L'administration peut être effectuée par voie nasale, orale ou parentérale.

Par voie orale ou par voie nasale, il est préféré d'administrer le traitement de façon répétée selon un rythme quotidien, biquotidien, hebdomadaire ou à intervalles réguliers tous les 2 à 6 jours. La dose par administration peut être donnée en une seule fois ou de façon fragmentée sur plusieurs heures ou encore de façon continue pendant une période de temps de quelques heures.

Par voie parentérale, le traitement peut être administré soit en injection unique, soit en injections répétées selon un rythme quotidien, biquotidien, hebdomadaire ou à intervalles réguliers tous les 2 à 6 jours. Un rythme préféré de traitement par voie parentérale est le traitement en injection unique.

Les doses recommandées varient en fonction du composé choisi et de la voie d'administration. Par voie orale ou nasale, les doses par administration sont comprises entre 0,01 mg/kg et 100 mg/kg, de préférence entre 0,1 et 50 mg/kg. Plus particulièrement pour le tiludronate, les doses par administration sont comprises entre 0,5 et 50 mg/kg, de façon préférée entre 1 et 20 mg/kg. Par voie parentérale, les doses par administration sont comprises entre 0,001 et 50 mg/kg, de préférence entre 0,005 et 30 mg/kg. Plus particulièrement pour le tiludronate, les doses sont comprises entre 0,1 et 50 mg/kg, de façon préférée entre 0,25 et 25 mg/kg.

### Exemple

Un effectif de 216 poules de souche ISA Brown âgées de 15 à 17 semaines a été réparti en 3 groupes dans l'objectif d'évaluer les effets sur la production d'oeufs d'une administration unique par voie sous-cutanée d'acide tiludronique, sous la forme de son sel de sodium (tiludronate disodique).

Trois doses ont été comparées : 0, 1 et 10 mg/kg (quantité correspondante d'acide tiludronique/kg). L'acide tiludronique était administré sous forme de solutions aqueuses injectables de telle sorte que le volume injecté par oiseau soit de 1 ml par kilo de poids vif. Les solutions d'acide tiludronique étaient donc concentrées à 0,1 % et 1 % pour les doses de 1 et 10 mg/kg respectivement. Le groupe témoin recevait un placebo correspondant à de l'eau pour préparation injectable. Les injections ont été pratiquées en région sous-cutanée au-dessus du muscle pectoral superficiel.

Les poules étaient hébergées en cages individuelles dans un même bâtiment à température et hygrométrie contrôlées. Chaque animal était identifié par un numéro porté sur une bague fixée à une aile. Elles ont reçu le même aliment standard pour poule pondeuse ad libitum pendant toute la durée de l'étude. L'eau de boisson était également distribuée ad libitum.

L'âge moyen d'entrée en ponte pour les poules de cette souche est de 20 semaines, avec des extrêmes compris entre 18 et 23 semaines. Ceci signifie pour les poules de cette étude que le délai moyen entre le traitement et l'âge d'entrée en ponte devait être de 3 à 5 semaines. La durée de l'étude a été de 40 semaines. En fin d'étude, les poules étaient âgées de 55 semaines.

La moitié de l'effectif a été sacrifié à la ponte du premier oeuf en vue de réaliser un examen histomorphométrique. Cet examen a été réalisé sur un des 2 os tarsométatarses.

L'autre moitié de l'effectif a été suivi afin de collecter les paramètres de ponte suivants : âge à la ponte du premier oeuf et nombre total d'oeufs pondus jusqu'à 55 semaines. Pour chaque groupe, le nombre moyen d'oeufs pondus par poule et par semaine a été calculé sur l'ensemble de la période de ponte et semaine par semaine afin d'établir les courbes de ponte.

Par ailleurs, une analyse de la résistance mécanique de la coquille et de l'épaisseur de la partie minéralisée de la coquille a été conduite à partir d'un échantillon de 12 poules aux deux échéances suivantes : à l'âge de 23 semaines (soit au début de la ponte) et à l'âge de 54 semaines. La résistance de la coquille était mesurée quantitativement par une technique de compression quasi-statique de la coquille de l'oeuf. L'épaisseur de la coquille était mesurée à partir d'images obtenues au microscope électronique.

A 55 semaines d'âge, tous les animaux ont été sacrifiés et un examen histomorphométrique d'un des os tarsométatarses a été réalisé dans les mêmes conditions que sur les os des poules sacrifiées à la ponte du premier oeuf.

Les critères « âge d'entrée en ponte », « nombre total d'oeufs pondus par poule », « nombre d'oeufs pondus par poule et par semaine sur l'ensemble de la ponte », « résistance mécanique de la coquille », « épaisseur de coquille » et « volume d'os médullaire » ont été comparés par une analyse de variance à 1 facteur (facteur dose).

### 1 - Variation de l'âge moyen d'entrée en ponte en fonction de la dose d'acide tiludronique

Le tableau 1 rapporte les âges moyens de ponte du premier oeuf en fonction de la dose d'acide tiludronique pour l'effectif de poules suivies jusqu'à l'âge de 55 semaines.

**Tableau 1 : Age à l'entrée en ponte (exprimé en jours) en fonction de la dose d'acide tiludronique (exprimée en mg/kg)**

| | | | |
|---|---|---|---|
| Dose d'acide tiludronique (mg/kg) | *0* | *1* | *10* |
| Age de l'entrée en ponte (jours) | 144,9 ± 8,1 | 148,3 ± 7,8 | 145,1 ± 8,8 |

Pour l'ensemble des poules, l'âge moyen d'entrée en ponte est d'environ 147 jours (soit 21 semaines d'âge), avec des extrêmes compris entre 124 jours (soit 17,7 semaines) et 165 jours (soit 23,6 semaines).

La période de ponte a été en moyenne de 34 semaines quel que soit le groupe. Les poules traitées à la dose de 1 mg/kg sont entrées en ponte en moyenne 3 jours après les poules des 2 autres groupes. Cependant, l'analyse statistique conclut à l'absence de différence significative (p = 0,11) entre les groupes.

Ainsi, le traitement par l'acide tiludronique n'a pas entrainé de modification de l'âge à l'entrée en ponte.

### 2 - Variation du nombre total d'oeufs pondus sur l'ensemble de la période de ponte en fonction de la dose d'acide tiludronique

Le nombre total d'oeufs pondus sur l'ensemble de la période de ponte est présenté dans le tableau 2.

**Tableau 2 : Nombre total moyen d'oeufs pondus par poule de l'entrée en ponte jusqu'à l'âge de 55 semaines, en fonction de la dose d'acide tiludronique (exprimé en mg/kg)**

| | | | |
|---|---|---|---|
| Dose d'acide tiludronique (mg/kg) | *0* | *1* | *10* |
| Nombre total moyen d'oeufs | **214,1 ± 14,0** | **221,6 ±11,2** | **219,8 ± 12,6** |

Il apparaît une différence sur le nombre moyen d'oeufs pondus par poule entre le groupe témoin et les 2 groupes traités par l'acide tiludronique : en moyenne, les poules traitées ont pondu entre 5,7 et 7,5 oeufs en plus. L'analyse statistique révèle une différence significative au seuil de 5 % entre les 3 groupes (p = 0,03). Une analyse 2 à 2 démontre que le nombre total d'oeufs pondus par poule dans le groupe traité à la dose de 1 mg/kg est significativement supérieur au nombre total d'oeufs pondus par poule dans le groupe témoin.

Le traitement par l'acide tiludronique a donc entrainé une augmentation du nombre d'oeufs pondus sur la période de ponte.

### 3 - Variation du nombre moyen d'oeufs pondus par semaine en fonction de la dose d'acide tiludronique

Le tableau 3, relatif au nombre moyen d'oeufs pondus par semaine, confirme cette observation.

**Tableau 3 : Nombre moyen d'oeufs pondus par poule et par semaine de l'entrée en ponte jusqu'à l'âge de 55 semaines, en fonction de la dose d'acide tiludronique (exprimée en mg/kg)**

| | | | |
|---|---|---|---|
| Dose d'acide tiludronique (mg/kg) | *0* | *1* | *10* |
| Nombre moyen/sem. | **6,23 ± 0,38** | **6,53 ± 0,27** | **6,39 ± 0,34** |

L'analyse statistique révèle une différence hautement significative (p = 0,001) entre les 3 groupes, le groupe traité à la dose de 1 mg/kg ayant pondu en moyenne par semaine de ponte plus d'oeufs que le groupe témoin.

### 4 - Courbe de ponte

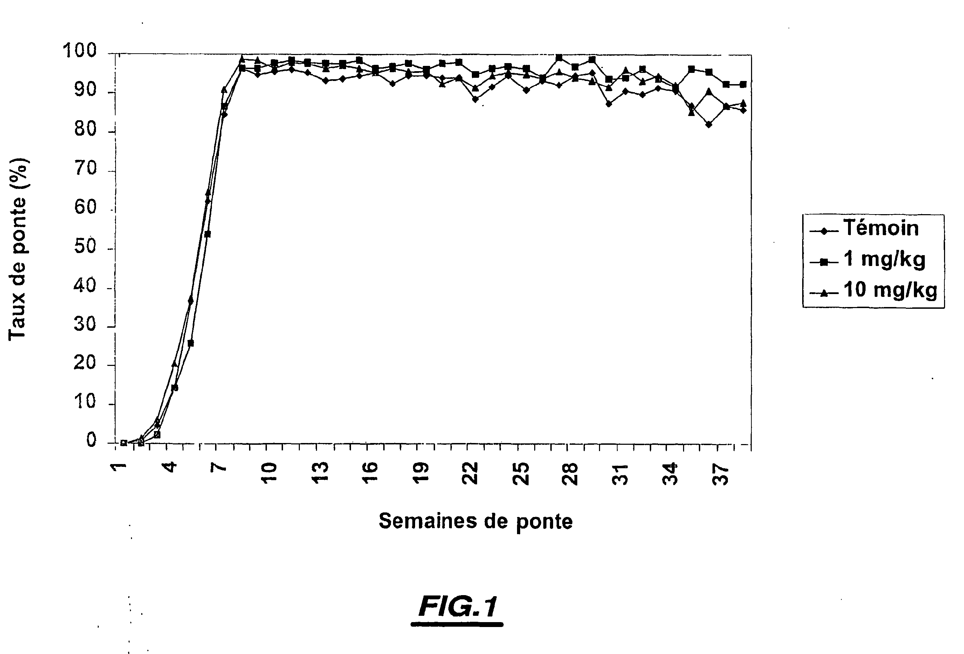

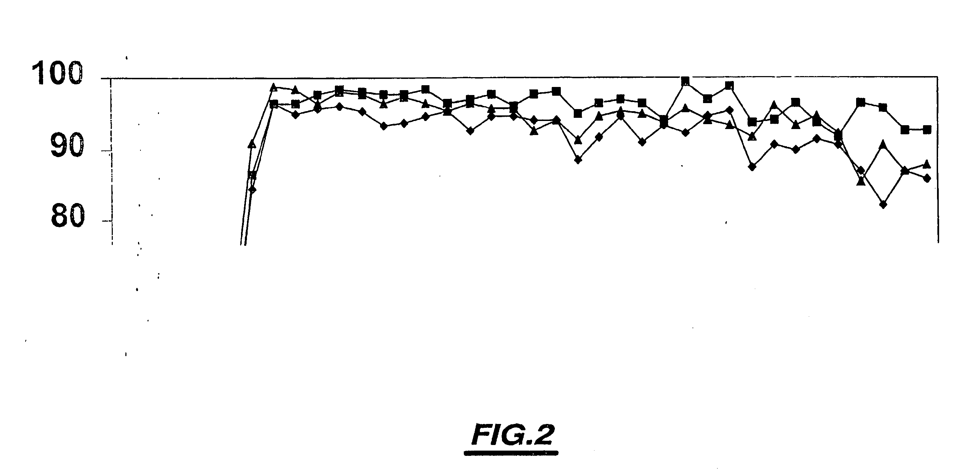

Les courbes de ponte construites à partir du nombre moyen d'oeufs pondus par poule et par semaine exprimé ensuite en pourcentage par rapport à la valeur théorique de 7 (soit un oeuf pondu par jour et par poule chaque semaine) sont présentées à la figure 1. La figure 2 est un agrandissement de la zone supérieure de la courbe de ponte. Le pic de ponte est plus élevé dans les groupes traités que dans le groupe témoin. La décroissance naturelle du taux de ponte au cours du temps est ralentie chez les poules traitées, en particulier à la dose de 1 mg/kg, comparativement aux témoins. La pente de la courbe de ponte apparaît donc sensiblement plus faible dans les groupes traités. Cet écart de pente est à l'origine des différences précédemment mises en évidence sur le nombre total d'oeufs pondus et sur le nombre moyen d'oeufs pondus par semaine.

### 5 - Résistance mécanique de la coquille d'oeuf

Les critères de qualité des coquilles des oeufs produits dans cette étude sont rapportés dans les tableaux 4 et 5.

Le tableau 4 présente les valeurs obtenues lors du test de résistance mécanique.

**Tableau 4 : Résistance mécanique (exprimée en N) de la coquille des oeufs prélevés sur un échantillon de 12 poules à 23 et 54 semaines d'âge, en fonction de la dose d'acide tiludronique (exprimée en mg/kg)**

| | | | |
|---|---|---|---|
| Dose d'acide *0* tiludronique (mg/kg) | | *1* | *10* |
| Résistance à 23 semaines | **37,2 ± 4,1** | **39,3 ± 5,2** | **41,5 ± 6,3** |
| Résistance à 54 semaines | **21,5 ± 10,1** | **28,3 ± 4,9** | **30,3 ± 6,1** |

Les résultats suggèrent une relation entre la dose et l'effet, aussi bien sur les oeufs prélevés en début de ponte qu'à l'âge de 54 semaines. L'écart entre le groupe témoin et les groupes traités est plus marqué à 54 semaines qu'à 23 semaines. Cela indique que la perte de résistance mécanique de la coquille des oeufs, processus naturel évoluant de façon progressive au fur et à mesure que l'on avance dans la période de ponte, est moindre chez les animaux traités avec l'acide tiludronique (environ - 28 % pour les 2 groupes traités entre les 2 échéances de mesure) que chez les témoins non traités (- 42 %). L'analyse statistique ne permet pas de révéler des écarts significatifs au seuil de 5 % ; ceci peut être expliqué par la variabilité individuelle relativement élevée qui caractérise ce paramètre.

L'examen du paramètre « épaisseur de la partie minéralisée de la coquille » présenté dans le tableau 5 permet de conclure dans le même sens que le critère précédent, même si l'analyse statistique ne permet pas de révéler des différences significatives au seuil de 5 %. La coquille apparaît plus épaisse chez les animaux traités par l'acide tiludronique ; l'effet est plus intense chez les poules traitées à la dose de 10 mg/kg, en particulier à l'âge de 54 semaines. Par ailleurs, il existe une bonne correspondance entre la résistance mécanique de la coquille et l'épaisseur de la partie minéralisée : plus l'épaisseur augmente plus la résistance est importante.

**Tableau 5 : Epaisseur de la partie minéralisée de la coquille (exprimée en mm) des oeufs prélevés sur un échantillon de 12 poules à 23 et 54 semaines d'âge, en fonction de la dose d'acide tiludronique (exprimée en mg/kg)**

| | | | |
|---|---|---|---|
| Dose d'acide tiludronique (mg/kg) | *0* | *1* | *10* |
| épaisseur à 23 semaines | **0,255** ± **0,028** | **0,256** ± **0,025** | **0,272** ± **0,013** |
| épaisseur à 54 semaines | **0,249 ± 0,015** | **0,267 ± 0,026** | **0,273 ± 0,021** |

Les 2 paramètres précédemment analysés sont de bons indicateurs de la qualité de la coquille. Les résultats obtenus dans cette étude suggèrent donc très fortement que l'acide tiludronique induit une amélioration de la qualité de la coquille de l'oeuf.

### 6 - Volume de l'os médullaire du tarsométatarse

A l'entrée en ponte les volumes osseux médullaires sont peu importants sans différence entre les groupes. Ceci correspond à la formation récente de l'os médullaire qui débute environ 2 semaines avant l'entrée en ponte, avec une forte variabilité individuelle.

A 55 semaines d'âge, il n'existe pas non plus d'écart significatif entre les groupes ; ceci peut s'expliquer également par la forte variabilité individuelle. Cependant, il apparaît que le développement de l'os médullaire est plus important chez les animaux ayant reçu le tiludronate que chez les témoins. Alors que chez ces derniers, le volume osseux médullaire a été multiplié en moyenne par un facteur 6, le facteur multiplicatif est de 9 à la dose de 1 mg/kg et de 13 à la dose de 10 mg/kg.

**Tableau 6 : Volume d'os médullaire (exprimé en %) de l'os tarsométatarse à l'entrée en ponte et à l'âge de 55 semaines, en fonction de la dose d'acide tiludronique (exprimée en mg/kg)**

| | | | |
|---|---|---|---|
| Dose d'acide tiludronique (mg/kg) | *0* | *1* | *10* |
| Entrée en ponte | **0,53 ± 0,76** | **0,31 ± 0,53** | **0,26 ± 0,33** |
| A 55 semaines d'âge | **2,93 ± 2,03** | **2,75 ± 2,72** | **3,53 ± 3,04** |

Ces résultats suggèrent un effet bénéfique de l'acide tiludronique sur le remodelage osseux médullaire. Cette observation va dans le sens de l'effet bénéfique précédemment rapporté sur la production d'oeufs et sur la qualité de la coquille de l'oeuf. Toutefois, en l'état actuel des connaissances sur le mode d'action des dérivés de l'acide bisphosphonique, cet effet bénéfique était inattendu.

## Revendications

1. Procédé pour augmenter la production d'oeufs et consolider la coquille des oeufs chez les volailles, comprenant l'administration à l'animal d'au moins un composé bisphosphonique choisi parmi un acide bisphosphonique, un sel physiologiquement acceptable de celui-ci , leurs hydrates et leurs mélanges et ceci avant l'âge moyen d'entrée en ponte.

2. Procédé selon la revendication 1, **caractérisé en ce que** le composé bisphosphonique a pour formule : 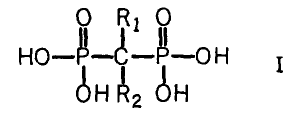
dans laquelle R₁ et R₂ sont indépendamment un atome d'hydrogène ; un atome d'halogène ; un groupe hydroxy ; un groupe -T ; ou un groupe -XT ;
T est choisi parmi un radical hydrocarboné aliphatique saturé ou insaturé, éventuellement substitué et/ou éventuellement interrompu par un ou plusieurs O, S, N, N-CO, CO-N, CO, SO ou SO₂ ; un radical carbocyclique ou hétérocyclique, saturé, insaturé ou aromatique, éventuellement substitué ; un radical présentant à la fois une partie aliphatique telle que définie ci-dessus et une partie carbocyclique et/ou hétérocyclique telle que définie ci-dessus, ledit radical étant éventuellement substitué et/ou éventuellement interrompu par un plusieurs O, S, N, N-CO, CO-N, CO, SO ou SO₂ ;
X est choisi parmi O, NH, NT, S, CO, CO-NT, NT-CO où T est tel que défini ci-dessus ;
ou est un sel ou hydrate physiologiquement acceptable de ce composé.

3. Procédé selon la revendication 2, **caractérisé en ce que** :
- R₁ représente un atome d'hydrogène, un atome d'halogène, un hydroxyle, un amino, un monoalkyl(C₁-C₄)amino, un dialkyl(C₁-C₄)amino ;
- R₂ représente un atome d'halogène, un alkyle linéaire comportant de 1 à 5 atomes de carbone non substitué ou substitué par un groupe choisi parmi un atome de chlore, un hydroxyle, un amino, un monoalkyl(C₁-C₄)amino, un dialkyl(C₁-C₄)amino ; un cycloalkyl(C₃-C₇)amino,
ou R₂ représente un phénoxy, un phényle, un thiol, un phénylthio, un chlorophénylthio, un pyridyle, un pyridylméthyle, un 1-pyridyl-1-hydroxyméthyle, un imidazolylméthyle, un thiomorpholin-4-yle.

4. Procédé selon la revendication 3, **caractérisé en ce que** le dérivé de l'acide bisphophonique est choisi parmi :
- l'acide 1-hydroxy-éthylidène bisphophonique et ses sels de sodium;
- l'acide 2-pyridin-2-yl-éthylidène bisphosphonique et ses sels de sodium ;
- l'acide dichlorométhylène bisphosphonique et ses sels de sodium ;
- l'acide 3-amino-1-hydroxy-propylidène bisphosphonique et ses sels de sodium ;
- l'acide 3-(diméthylamino)-1-hydroxypropylidène bisphosphonique et ses sels ;
- l'acide 1-hydroxy-3-(méthylpentylamino)propylidène bisphosphonique et ses sels ;
- l'acide 4-amino-1-hydroxy-butylidène bisphosphonique et ses sels de sodium ;
- l'acide 6-amino-1-hydroxy-hexylidène bisphosphonique et ses sels;
- l'acide phénoxyméthylène bisphosphonique et ses sels ;
- l'acide thiomorpholinométhylène bisphosphonique et ses sels ;
- l'acide 4-chloro-phénylthiométhylène bisphosphonique et ses sels ;
- l'acide 1-hydroxy-2-(pyridin-3-yl)éthylidène bisphosphonique et ses sels de sodium ;
- l'acide 1-hydroxy-2-(1H-imidazol-1-yl)éthylidène bisphosphonique et ses sels ;
- l'acide (cycloheptylamino)méthylène bisphosphonique et ses sels ;
- l'acide 2-hydroxyéthylidène-2-(pyridin-3-yl)-1,1-bisphosphonique et ses sels de sodium.

5. Procédé selon la revendication 4, **caractérisé en ce que** l'acide bisphosphonique est l'acide 4-chlorophénylthiométhylène bisphosphonique.

6. Procédé selon l'une quelconque des revendications précédentes, **caractérisé en ce que** l'animal est une volaille productrice d'oeufs destinés à la consommation.

7. Procédé selon la revendication 6, **caractérisé en ce que** la volaille est choisie parmi la poule, le canard et la caille.

8. Procédé selon l'une quelconque des revendications précédentes, **caractérisé en ce que** l'administration est réalisée par voie orale, parentérale ou nasale.

9. Procédé selon l'une quelconque des revendications précédentes, **caractérisé en ce que** la dose administrée par voie orale ou nasale est comprise entre environ 0,01 mg/kg et 100 mg/kg de poids corporel.

10. Procédé selon l'une quelconque des revendications précédentes, **caractérisé en ce que** la dose administrée par voie parentérale est comprise entre environ 0,005 et 30 mg/kg de poids corporel.

11. Procédé selon l'une quelconque des revendications précédentes, **caractérisé en ce que** l'administration du composé bisphosphonique est initiée dans les six semaines précédant l'âge moyen d'entrée en ponte.

12. Procédé selon la revendication précédente, **caractérisé en ce que** les dérivés de l'acide bisphosphonique sont administrés en une seule fois ou à intervalles avant l'entrée en ponte.

## Claims

1. Process for increasing egg production and strengthening eggshells in poultry, which comprises the administration to the animal of at least one bisphosphonic compound selected from a bisphosphonic acid, a physiologically acceptable salt thereof, hydrates thereof and mixtures thereof, and this before the average age of the onset of laying.

2. Process according to claim 1, **characterised in that** the bisphosphonic compound has the formula: 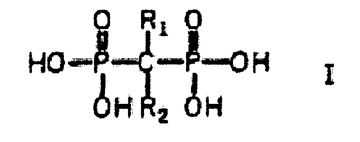
in which R₁ and R₂ are, independently selected from a hydrogen atom; a halogen atom; a hydroxy group; a group -T; and a group -XT;
T is selected from an aliphatic saturated or unsaturated hydrocarbon-based radical, optionally substituted and/or optionally interrupted with one or more O, S, N, N-CO, CO-N, CO, SO or SO₂; a saturated, unsaturated or aromatic, optionally substituted carbocyclic or heterocyclic radical; a radical with both an aliphatic part as defined above and a carbocyclic and/or heterocyclic part as defined above, the said radical being optionally substituted and/or optionally interrupted with a plurality of O, S, N, N-CO, CO-N, CO, SO or SO₂;
X is selected from O, NH, NT, S, CO, CO-NT and NT-CO, in which T is as defined above; or is a physiologically acceptable salt or hydrate of this compound.

3. Process according to claim 2, **characterised in that**:
- R₁ represents a hydrogen atom, a halogen atom, a hydroxyl, an amino, a mono(C₁-C₄)alkylamino, a di(C₁-C₄)alkylamino;
- R₂ represents a halogen atom, a linear alkyl comprising from 1 to 5 carbon atoms which is unsubstituted or substituted with a group chosen from a chlorine atom, a hydroxyl, an amino, a mono(C₁-C₄)alkylamino, a di(C₁-C₄)alkylamino; a (C₃-C₇)cycloalkylamino,
or R₂ represents a phenoxy, a phenyl, a thiol, a phenylthio, a chlorophenylthio, a pyridyl, a pyridylmethyl, a 1-pyridyl-1-hydroxymethyl, an imidazolylmethyl and a 4-thiomorpholinyl.

4. Process according to claim 3, **characterised in that** the bisphosphonic acid derivative is selected from:
- 1-hydroxyethylidene bisphosphonic acid and its sodium salts;
- 2-pyridin-2-ylethylidene bisphosphonic acid and its sodium salts;
- dichloromethylene bisphosphonic acid and its sodium salts;
- 3-amino-1-hydroxypropylidene bisphosphonic acid and its sodium salts;
- 3-(dimethylamino)-1-hydroxypropylidene bisphosphonic acid and its salts;
- 1-hydroxy-3-(methylpentylamino)propylidene bisphosphonic acid and its salts;
- 4-amino-1-hydroxybutylidene bisphosphonic acid and its sodium salts;
- 6-amino-1-hydroxyhexylidene bisphosphonic acid and its salts;
- phenoxymethylene bisphosphonic acid and its salts;
- thiomorpholinomethylene bisphosphonic acid and its salts;
- 4-chlorophenylthiomethylene bisphosphonic acid and its salts,
- 1-hydroxy-2-(pyridin-3-yl)ethylidene bisphosphonic acid and its sodium salts;
- 1-hydroxy-2-(1H-imidazol-1-yl)ethylidene bisphosphonic acid and its salts;
- (cycloheptylamino)methylene bisphosphonic acid and its salts;
- 2-hydroxyethylidene-2-(pyridin-3-yl)-1,1-bisphosphonic acid and its sodium salts.

5. Process according to claim 4, **characterised in that** the bisphosphonic acid is 4-chlorophenylthiomethylene bisphosphonic acid.

6. Process according to any one of the preceding claims, **characterised in that** the animal is poultry which produces eggs intended for consumption.

7. Process according to claim 6, **characterised in that** the poultry is chosen from hens, ducks and quails.

8. Process according to any one of the preceding claims, **characterised in that** the administration is carried out via the oral, parenteral or nasal route.

9. Process according to any one of the preceding claims, **characterised in that** the dose administered via the oral or nasal route is between about 0.01 and 100 mg/kg of body weight.

10. Process according to any one of the preceding claims, **characterised in that** the dose administered via the parenteral route is between about 0.005 and 30 mg/kg of body weight.

11. Process according to any one of the preceding claims, **characterised in that** the administration of the bisphosphonic compound is initiated in the six weeks preceding the average age of onset of laying.

12. Process according to the preceding claim, **characterised in that** the bisphosphonic acid derivatives are administered once only or at intervals before the onset of laying.

## Patentansprüche

1. Verfahren zur Erhöhung der Eierproduktion und Verfestigung von Eierschalen bei Geflügel, umfassend die Verabreichung von mindestens einer Bisphosphonverbindung, die ausgewählt ist aus einer Bisphosphonsäure, einem physiologisch verträglichen Salz davon, deren Hydraten und deren Mischungen, an ein Tier vor dem Durchschnittsalter des Beginns der Legezeit.

2. Verfahren nach Anspruch 1, **dadurch gekennzeichnet, dass** die Bisphosphonverbindung die Formel: 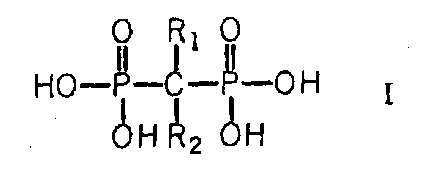 besitzt,
worin R₁ und R₂ unabhängig voneinander ein Wasserstoffatom; ein Halogenatom; eine Hydroxygruppe; eine -T-Gruppe; oder eine -XT-Gruppe sind;
T ausgewählt ist aus einem gesättigten oder ungesättigten aliphatischen Kohlenwasserstoffrest, der gegebenenfalls mit einem oder mehreren von O, S, N, N-CO, CO-N, CO, SO oder SO₂ substituiert ist und/oder von diesen gegebenenfalls unterbrochen wird; einem gesättigten, ungesättigten oder aromatischen, carbocyclischen oder heterocyclischen Rest, der gegebenenfalls substituiert ist; einem Rest, der zugleich einen aliphatischen Teil, wie er oben definiert ist, und einen carbocyclischen und/oder heterocyclischen Teil, wie er oben definiert ist, aufweist, wobei der Rest gegebenenfalls mit einem oder mehreren von O, S, N, N-CO, CO-N, CO, SO oder SO₂ substituiert ist und/oder von diesen gegebenenfalls unterbrochen wird;
X ausgewählt ist aus O, NH, NT, S, CO, CO-NT, NT-CO oder T, das wie oben definiert ist;
oder ein physiologisch verträgliches Salz oder Hydrat der Verbindung ist.

3. Verfahren nach Anspruch 2, **dadurch gekennzeichnet, dass**:
- R₁ ein Wasserstoffatom, ein Halogenatom, ein Hydroxyl, ein Amino, ein Monoalkyl(C₁-C₄)amino, ein Dialkyl(C₁-C₄)amino ist;
- R₂ ein Halogenatom, ein lineares Alkyl mit 1 bis 5 Kohlenstoffatomen, das nicht substituiert oder mit einer Gruppe, die ausgewählt ist aus einem Chloratom, einem Hydroxyl, einem Amino, einem Monoalkyl(C₁-C₄)amino, einem Dialkyl(C₁-C₄)amino; einem Cycloalkyl(C₃-C₇)amino, substituiert ist, ist,
oder R₂ ein Phenoxy, ein Phenyl, ein Thiol, ein Phenylthio, ein Chlorphenylthio, ein Pyridyl, ein Pyridylmethyl, ein 1-Pyridyl-1-hydroxymethyl, ein Imidazolylmethyl, ein Thiomorpholin-4-yl ist.

4. Verfahren nach Anspruch 3, **dadurch gekennzeichnet, dass** das Bisphosphonsäurederivat ausgewählt ist aus:
- 1-Hydroxyethylidenbisphosphonsäure und deren Natriumsalzen;
- 2-Pyridin-2-ylethylidenbisphosphonsäure und deren Natriumsalzen;
- Dichlormethylenbisphosphonsäure und deren Natriumsalzen;
- 3-Amino-1-hydroxypropylidenbisphosphonsäure und deren Natriumsalzen;
- 3-(Dimethylamino)-1-hydroxypropylidenbisphosphonsäure und deren Salzen;
- 1-Hydroxy-3-(methylpentylamino)propylidenbisphosphonsäure und deren Salzen;
- 4-Amino-1-hydroxybutylidenbisphosphonsäure und deren Natriumsalzen;
- 6-Amino-1-hydroxyhexylidenbisphosphonsäure und deren Salzen;
- Phenoxymethylenbisphosphonsäure und deren Salzen;
- Thiomorpholinomethylenbisphosphonsäure und deren Salzen;
- 4-Chlorphenylthiomethylenbisphosphonsäure und deren Salzen;
- 1-Hydroxy-2-(pyridin-3-yl)ethylidenbisphosphonsäure und deren Natriumsalzen;
- 1-Hydroxy-2-(1H-imidazol-1-yl)ethylidenbisphosphonsäure und deren Salzen;
- (Cycloheptylamino)methylenbisphosphonsäure und deren Salzen;
- 2-Hydroxyethyliden-2-(pyridin-3-yl)-1, 1-bisphosphonsäure und deren Natriumsalzen.

5. Verfahren nach Anspruch 4, **dadurch gekennzeichnet, dass** die Bisphosphonsäure 4-Chlorphenylthiomethylenbisphosphonsäure ist.

6. Verfahren nach irgendeinem der vorhergehenden Ansprüche, **dadurch gekennzeichnet, dass** das Tier ein Geflügel ist, das-zum Verzehr bestimmte Eier produziert.

7. Verfahren nach Anspruch 6, **dadurch gekennzeichnet, dass** das Geflügel ausgewählt ist aus Huhn, Ente und Wachtel.

8. Verfahren nach irgendeinem der vorhergehenden Ansprüche, **dadurch gekennzeichnet, dass** die Verabreichung auf oralem, parenteralem oder nasalem Wege erfolgt.

9. Verfahren nach irgendeinem der vorhergehenden Ansprüche, **dadurch gekennzeichnet, dass** die auf oralem oder nasalem Wege verabreichte Dosis zwischen ungefähr 0,01 mg/kg und 100 mg/kg Körpergewicht umfasst.

10. Verfahren nach irgendeinem der vorhergehenden Ansprüche, **dadurch gekennzeichnet, dass** die auf parenteralem Wege verabreichte Dosis zwischen ungefähr 0,005 und 30 mg/kg Körpergewicht umfasst.

11. Verfahren nach irgendeinem der vorhergehenden Ansprüche, **dadurch gekennzeichnet, dass** die Verabreichung der Bisphosphonverbindung innerhalb der sechs Wochen vor dem Durchschnittsalter des Beginns der Legezeit begonnen wird.

12. Verfahren nach dem vorhergehenden Anspruch, **dadurch gekennzeichnet, dass** die Bisphosphonsäurederivate auf einmal oder mit Zeitabständen vor Beginn der Legezeit verabreicht werden.
